Europäisches Patentamt

European Patent Office

Office européen des brevets

(19)

(11) EP 0 524 604 B1

(12) EUROPÄISCHE PATENTSCHRIFT

(45) Veröffentlichungstag und Bekanntmachung des Hinweises auf die Patenterteilung:
14.10.1998 Patentblatt 1998/42

(51) Int Cl.⁶: **C12N 15/55**, C12P 41/00, C12P 7/40, C12N 1/21 // (C12N1/21, C12R1:19)

(21) Anmeldenummer: 92112446.7

(22) Anmeldetag: 21.07.1992

(54) **Gentechnologisches Verfahren zur Herstellung von S-(+)-2,2-Dimethylcyclopropancarboxamid mittels Mikroorganismen**

Process for the preparations of S-(+)-2,2-dimethyl-cyclopropane carboxamid by genetically engineered microorganismes

Procédé de préparation par genic génétique de l'amide d'acide S-(+)-2,2-diméthyl cyclopropane carboxylique avec des microorganismes

(84) Benannte Vertragsstaaten:
**AT BE CH DE DK ES FR GB IT LI LU NL SE**

(30) Priorität: **26.07.1991 CH 2247/91**

(43) Veröffentlichungstag der Anmeldung:
**27.01.1993 Patentblatt 1993/04**

(73) Patentinhaber: **LONZA A.G.**
**CH-3945 Gampel/Wallis (CH)**

(72) Erfinder:
 • **Zimmermann, Thomas, Dr.**
  **Naters (Kanton Wallis) (CH)**
 • **Robins, Karen**
  **Visp (Kanton Wallis) (CH)**
 • **Birch, Olwen M.**
  **Naters (Kanton Wallis) (CH)**
 • **Böhlen, Elisabeth**
  **Naters (Kanton Wallis) (CH)**

(74) Vertreter: **Weinhold, Peter, Dr. et al**
**Winter, Brandl, Fürniss, Hübner, Röss, Kaiser, Polte, Kindermann Partnerschaft**
**Patent- und Rechtsanwaltskanzlei**
**Alois-Steinecker-Strasse 22**
**85354 Freising (DE)**

(56) Entgegenhaltungen:
 **EP-A- 0 416 282    EP-A- 0 433 117**
 **EP-A- 0 502 525**

 • CHEMICAL ABSTRACTS, vol. 103, no. 15, 14. Oktober 1985, Columbus, Ohio, US; abstract no. 123058c, SUMITOMO 'Optically active 2,2-dimethylcyclopropanecarboxylic acids.' Seite 688 ; & JP-A-60 056 936 (SUMITOMO) 2. April 1985
 • CHEMICAL ABSTRACTS, vol. 95, no. 19, 9. November 1981, Columbus, Ohio, US; abstract no. 167131s, ASAHI CHEMICAL 'Manufacture of 7-aminocepham compounds.' Seite 563 ; & JP-A-56 085 298 (ASAHI CHEMICAL) 11 Juli 1981

**Beschreibung**

Die Erfindung betrifft ein neues Verfahren zur Herstellung von S-(+)-2,2-Dimethylcyclopropancarboxamid mit neuen für das Verfahren geeignetenMikroorganismen. Diese Mikroorganismen sind mit einem neuen Gen, das eine stereospezifische Hydrolase bildet, transformiert und damit befähigt im racemischen R,S-($\pm$)-2,2-Dimethylcyclopropancarboxamid das R-(-)-Isomere in die entsprechende Säure zu biotransformieren, wobei optisch aktives S-(+)-2,2-Dimethylcyclopropancarboxamid anfällt.

Im folgenden wird 2,2-Dimethylcyclopropancarboxamid mit 2,2-DMCPCA und 2,2-Dimethylcyclopropancarbonsäure mit 2,2-DMCPCS abgekürzt.

Optisch reines S-(+)-2,2-DMCPCA dient als Ausgangsmaterial zur Herstellung des Dehydropeptidase-Inhibitors Cilastatin, welcher in der Therapie zusammen mit Penem- bzw. Carbapenem-Antibiotika verabreicht wird, um die Desaktivierung der Antibiotika durch eine renale Dehydropeptidase in der Niere zu verhindern (Lit: EP 048 301).

Beispiele von Mikroorganismen, die eine stereospezifische Hydrolase für das R-(-)-2,2-DMCPCA bilden sind Mikroorganismen der Spezies Comamonas acidovorans A:18 (DSM-Nr. 6351), Bakterium sp VIII:II (DSM-Nr. 6316), Pseudomonas sp. NSAK:42 (DSM-Nr. 6433) und Comamonas acidovorans TG 308 (DSM-Nr. 6552) sowie deren Deszendenten und Mutanten. Diese Mikroorganismen sind bereits ausführlich in der Europäischen Patentanmeldung 92103780.0 beschrieben.

Die Aufgabe der vorliegenden Erfindung bestand darin, mittels rekombinanten DNA-Techniken neue Mikroorganismen als Produktionsstämme zur Verfügung zu stellen, in denen die katalytische Fähigkeit sowie die Expression dieses Hydrolase-Gens gegenüber dem bekannten Verfahren erheblich gesteigert werden kann.

Erfindungsgemäss wurde diese Aufgabe mit einer neuen DNA, die für das Hydrolase-Gen codiert, mit neuen Hybridplasmiden, enthaltend diese DNA, mit neuen Mikroorganismen, die mit diesem Hybridplasmid transformiert sind und mit einem neuen Verfahren gelöst.

Fig. 1    zeigt die Restriktionskarte des Gens.
Fig. 2    zeigt ein Schema des Hybridplasmids pCAR6.
Fig. 3    zeigt sowohl die Aminosäure- als auch die DNA-Sequenz des Gens, die für die stereospezifische Hydrolase codiert.

Erfindungsgemäss wird das Verfahren derart durchgeführt, dass man im racemischen R,S-($\pm$)-2,2-DMCPCA das R-(-)-2,2-DMCPCA mittels Mikroorganismen, die mit einem Gen, das eine stereospezifische Hydrolase bildet, transformiert sind, zur R-(-)-2,2-DMCPCS biotransformiert, wobei optisch aktives S-(+)-2,2-DMCPCA anfällt, und dieses isoliert.

Herstellung der transformierten Mikroorganismen

Die Herstellung der erfindungsgemässen Mikroorganismen, die eine stereospezifische Hydrolase bilden, kann derart erfolgen, dass man

a) eine für die erfindungsgemässe Hydrolase codierende DNA isoliert
b) diese spezifische Gensequenz in einen Expressionsvektor einführt, wobei ein Hybridplasmid entsteht, wobei es gegebenenfalls vorteilhaft sein kann, im Hybridplasmid weitere Modifikationen vorzunehmen, die eine effektivere Expression ermöglichen
c) dieses Hybridplasmid mittels Transformation in einen für das Verfahren geeigneten
d) Mikroorganismus (Wirtsstamm) einbringt (Transformation e)), wobei dieser transformierte Mikroorganismus dann
f) den Produktionsstamm für das erfindungsgemässe Verfahren (Biotransformation g)) bildet.

a) Isolation der stereospezifischen Hydrolase-DNA

Als Quelle der Hydrolase-DNA, die im folgenden als Hydrolase DNA (rad) bzw. Hydrolase-Gen (rad) bezeichnet wird, kann beispielsweise die chromosomale DNA der Mikroorganismen Comamonas acidovorans A:18 (DSM-Nr. 6315) oder Comamonas acidovorans TG 308 (DSM-Nr. 6552), die bereits in der Europäischen Patentanmeldung 92103780.0 beschrieben sind, dienen.

Vorzugsweise dient als Quelle Comamonas acidovorans A:18. Die Hydrolase-DNA kann aus einer linearen Genbank von Comamonas acidovorans A:18 in Escherichia coli (E.coli) XL1-Blue[R] mit BLUESCRIPT[R] (BLUESCRIPT-KS+ oder BLUESCRIPT-SK+)(erhältlich von Stratagene Co., Lieferant Genofit SA, Genf) als handelsüblicher Genbank-Vektor isoliert werden.

Dazu wird beispielsweise zunächst die chromosomale DNA von <u>Comamonas</u> <u>acidovorans</u> A:18 in Anlehnung an die Methode von R.H.Chesney et al., (J.Mol.Biol. <u>130,</u> (1979), S.161-173) isoliert. Diese DNA kann dann mittels üblichen molekularbiologischen Methoden mit dem Restriktionsenzym EcoRI geschnitten und anschliessend in die zuvor gleichermassen geschnittene Expressionsvektor-DNA pBLUESCRIPT-KS+$^R$ inseriert werden. Anschliessend kann diese ligierte DNA (Hybridplasmid-Gemisch) beispielsweise nach der Methode von S.Fiedler und R.Wirth (Analyt.Biochem. <u>170</u> (1988), S.38-44) in die kompetenten handelsüblichen Mikroorganismen <u>E.</u> <u>coli</u> XL1-Blue$^R$ transformiert werden.

Das "Screening" der Genbank kann ebenfalls nach an sich bekannten Methoden erfolgen. Hierzu werden die Hybridplasmid-Klone zweckmässig in einem geeigneten Medium mit R,S-(±)2,2-DMCPCA als einzige N-Quelle, einer üblichen C-Quelle, einem geeigneten Induktor und einem geeigneten Antibiotikum auf ihre Wachstumsfähigkeit überprüft. Mit diesem "Screening" werden dann zweckmässig die Hybridplasmid-Klone selektiert, die das aktive Hydrolase-Gen <u>(rad)</u> auf der Hybridplasmid-DNA enthalten und damit befähigt sind, vorzugsweise das R-(-)-2,2-DMCPCA als einzige N-Quelle zu nutzen. Diese Hybridplasmid-Klone sind dann in der Lage,das R-(-)-2,2-DMCPCA zur entsprechenden Säure zu hydrolysieren.

Die Lokalisation des Hydrolase-Gens <u>(rad)</u> erfolgt dann zweckmässig in dem Hybridplasmid pCAR1 (ausgewählt aus den Hybridplasmid-Klonen), welches aus dem Expressionsvektor pBLUESCRIPT-KS+$^R$ und einem EcoRI- "Insert" von ca. 23 kb besteht.

Die eigentliche Lokalisation des Hydrolase-Gens <u>(rad)</u> erfolgt dann zweckmässig mit der an sich bekannten "Southern-Blot"-Hybridisierung. (Current Protocols in Molecular Biology, John Wiley and Sons, New York, 1987, Abschnitt 2.9). Dazu wird zunächst zweckmässig Hybridplasmid pCAR1 mit den Restriktionsenzymen BamHI, PstI, PvuII und EcoRI verdaut. Die dabei entstandenen DNA-Fragmente werden dann zweckmässig gegen radioaktiv markierte DNA-Oligomere, die der N-terminalen Proteinsequenz der Hydrolase entsprechen, hybridisiert. Auf diese Weise kann ein 2,3 kb grosser EcoRI-BamHI-DNA-Abschnitt bzw. ein 1,85 kb grosser PvuII-BamHI-DNA-Abschnitt auf dem Hybridplasmid pCAR1 markiert werden.

Die DNA-Oligomere für die Hybridisierung können nach fachmännisch üblichen Methoden erhalten werden. Beispielsweise indem man die stereospezifische Hydrolase chromatographisch anreichert, die N-terminalen Aminosäuren bestimmt und dann die entsprechende DNA-Sequenz synthetisiert und radioaktiv markiert.

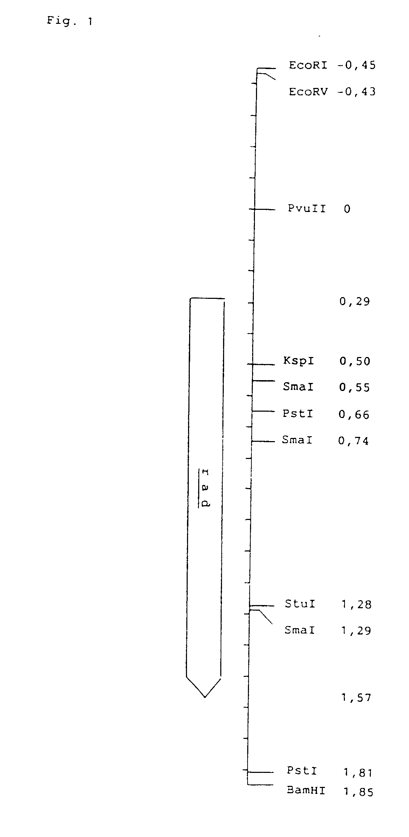

Der nun bekannte DNA-Abschnitt, der für die stereospezifische Hydrolase <u>(rad)</u> codiert und dessen Restriktionskarte in Fig. 1 charakterisiert ist, ist ebenso Bestandteil der Erfindung und kann dann mit den Restriktionsenzymen BamHI und EcoRI bzw. BamHI und PvuII aus dem Hybridplasmid pCAR1 nach fachmännisch üblichen Methoden isoliert werden, um u.a. nach Analyse der Nukleotidsequenz über den genetischen Code die gesamte Aminosäuresequenz zu bestimmen und um die für das Verfahren geeigneten transformierten Mikroorganismen herzustellen.

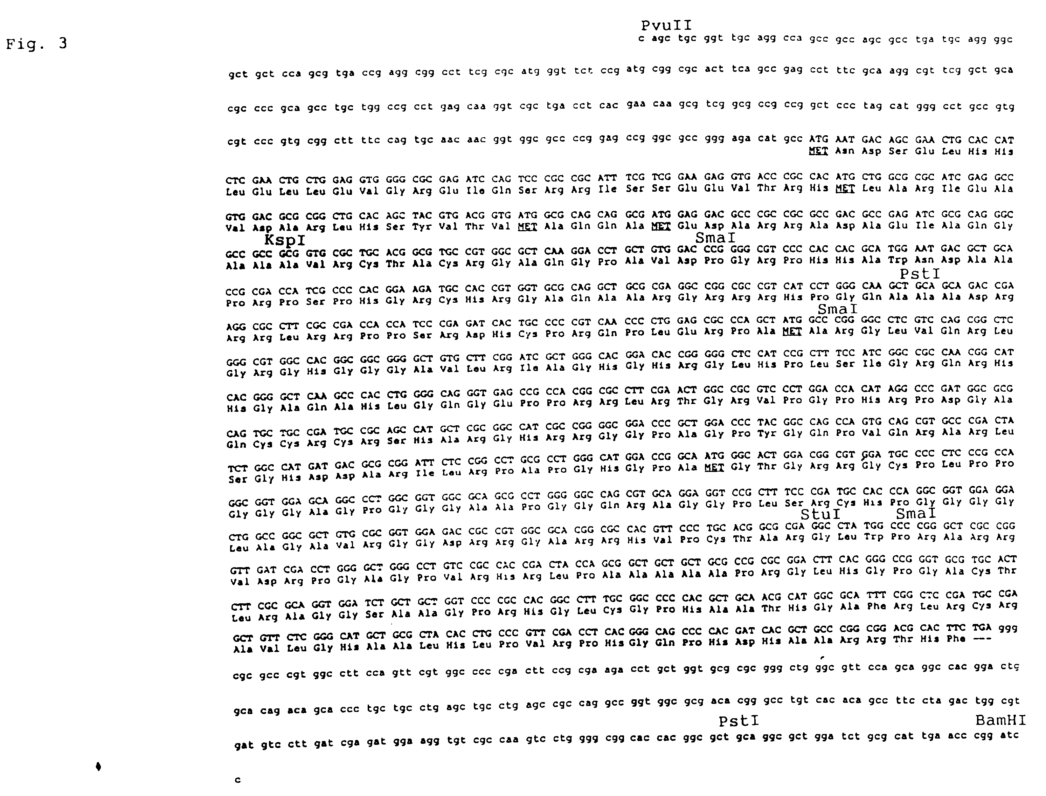

Bestandteil der Erfindung ist demnach auch sowohl ein DNA-Fragment, das für ein Polypeptid mit stereospezifischer Hydrolase-Aktivität, dessen Aminosäuresequenz in Fig. 3 dargestellt ist, codiert, als auch ein DNA-Fragment, welches mit dem in Fig. 3 dargestellten DNA-Fragment hybridisiert und für dieses Polypeptid codiert.

b) <u>Ligation der spezifischen Gensequenz (Hydrolase-Gen; rad) in Expressionsvektoren</u>

Die so erhaltene Gensequenz kann mittels üblicher molekularbiologischer Techniken mit einer zuvor gleichermassen geschnittenen Expressionsvektor-DNA zu einem Hybridplasmid ligiert werden.

Expressionsvektoren enthalten üblicherweise einen geeigneten, meist regulierbaren Promotor (Expressionskontrollsequenz). Hinter diesem Promotor liegen günstigerweise in Transkriptionsrichtung eine oder mehrere singuläre Schnittstellen für Restriktionsenzyme. In diese Schnittstellen wird dann üblicherweise der gewünschte Genabschnitt inseriert, an dessen Expression man interessiert ist.

Für das erfindungsgemässe Verfahren können entweder Expressionsvektoren mit breitem Wirtsspektrum ("broad host range") angewendet werden, oder es kann beispielsweise der handelsübliche Expressionsvektor pBLUESCRIPT-KS+$^R$ angewendet werden. Vorzugsweise wird als Expressionsvektor pBLUESCRIPT-KS+$^R$ mit dem Promotor P$_{Lac}$ (Lactose-Promotor) angewendet.

Zweckmässig wird Expressionsvektor pBLUESCRIPT-KS+$^R$ mit den Restriktionsenzymen EcoRI und BamHI oder mit PvuII und BamHI geschnitten und die entstandenen Restriktionsenden mit dem isolierten DNA-Abschnitt (EcoRI-BamHI oder PvuII-BamHI), welcher für die stereospezifische Hydrolase codiert, mittels beispielsweise T4-DNA-Ligase ligiert.

c) <u>Hybridplasmide</u>

Die Erfindung betrifft weiterhin die so entstandenen Hybridplasmide, die die stereospezifische Hydrolase-Gensequenz <u>(rad)</u> enthalten.

Grundsätzlich sind alle Hybridplasmide geeignet, welche die für die erfindungsgemässe Hydrolase kodierende DNA-Sequenz in dem gewählten Mikroorganismus (Produktionsstamm) zu replizieren und exprimieren vermögen.

Geeignete Hybridplasmide enthalten von ihrem ursprünglichen Expressionsvektor ein intaktes Replikon und ein Markierungsgen, welches die Selektion und die Identifizierung der mit dem Hybridplasmid transformierten Mikroorganismen aufgrund eines phänotypischen Merkmals ermöglicht. Ein geeignetes Markierungsgen verleiht dem Mikroorganismus beispielsweise Resistenz gegenüber Antibiotika.

Um eine effektive Expression in einem Hybridplasmid zu erreichen, ist es zweckmässig, dass das Hydrolase-Gen (rad) richtig (in "Phase") mit dem Promotor angeordnet ist.

Beispiele für solche Hybridplasmide, die zur Expression des Hydrolase-Gens in einem E.coli-Stamm geeignet sind, sind Hybridplasmid pCAR5 und pCAR6, mit dem Markierungsgen bla (verleiht Resistenz gegenüber Ampicillin; Ap$^R$) und dem Promotor P$_{Lac}$. Zweckmässig besteht Hybridplasmid pCAR5 aus dem 2,3 kb grossen EcoRI-BamHI-DNA-Fragment (Restriktionskarte Fig. 1) und Expressionsvektor pBLUESCRIPT-KS+. Hybridplasmid pCAR6 besteht zweckmässig aus dem 1,85 kb grossen PvuII-BamHI-DNA-Fragment Fig. 1 (Restriktionskarte) und Expressionsvektor pBLUESCRIPT-KS+.

Zweckmässig wird das Hybridplasmid pCAR6 mit dem Promotor P$_{Lac}$ angewendet, wobei die Expression des Hydrolase-Gens (rad) je nach Wirtsstamm mit Isopropylthiogalactosid (IPTG) induziert wird.

Das Hybridplasmid pCAR6 wurde sowohl am 6.06.1991 unter der DSM-Nr. 6551 in E.coli XL1-Blue$^R$ als auch am 21.04.1992 unter der DSM-Nr. 7053 in E.coli DH5, bei der Deutschen Sammlung für Mikroorganismen und Zellkulturen GmbH, Mascheroderweg 1b, D-3300 Braunschweig, hinterlegt.

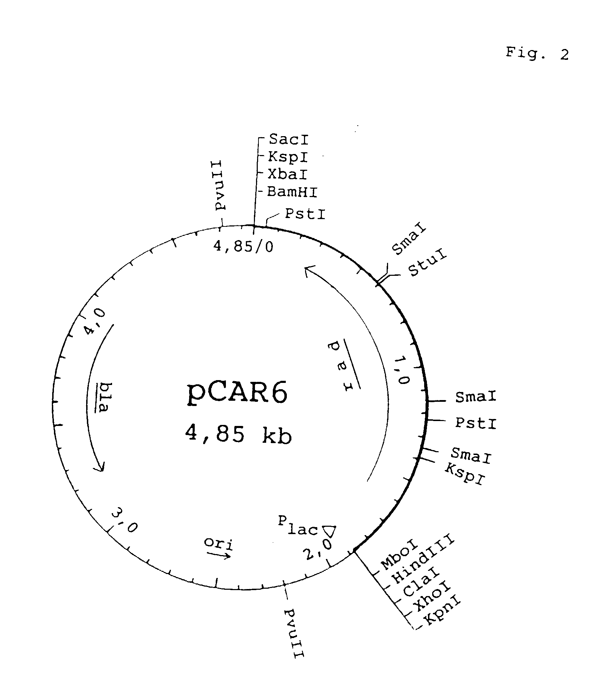

Fig. 2 zeigt ein Schema des Hybridplasmids pCAR6.

d) Wirtsstämme

Die so erhaltenen Hybridplasmide werden zweckmässig in Wirtsstämme eingesetzt.

Im Falle von "broad-host-range" Hybridplasmiden (Hybridplasmide mit breitem Wirtsspektrum), werden zweckmässig Wirtsstämme mit hoher Substrat- und Edukttoleranz eingesetzt, wie z.B. Mikroorganismen der Gattung Pseudomonas, Comamonas, Acinetobacter, Rhizobium, Agrobacterium oder Escherichia.

Im Falle von Hybridplasmiden, die ein enges Wirtsspektrum aufweisen, wie beispielsweise Hybridplasmid pCAR6, werden üblicherweise die spezifischen Wirtsstämme eingesetzt, in denen sie replizieren.

Vorzugsweise werden demnach als Wirtsstämme für Hybridplasmid pCAR6 Mikroorganismen der Gattung Escherichia angewendet, insbesondere die der Spezies E.coli, die in Tabelle 1 aufgeführt sind.

e) Transformation

Die Transformation der Wirtsstämme mit den erfindungsgemässen Hybridplasmiden erfolgt nach bekannten Verfahren. Die Isolierung der transformierten Wirtsstämme erfolgt dann vorzugsweise aus einem selektiven Nährmedium, dem das Antibiotikum zugesetzt wird, gegen welches das im Hybridplasmid enthaltene Markierungsgen Resistenz verleiht. Wenn bevorzugt das Hybridplasmid pCAR6 eingesetzt wird, welches das bla-Gen enthält, wird demgemäss dem Nährmedium Ampicillin zugesetzt.

f) Produktionsstamm

Als Produktionsstämme können erfindungsgemäss alle Wirtsstämme dienen, die mit einem Hybridplasmid, welches das stereospezifische Hydrolase-Gen enthält, transformiert sind. Vorzugsweise dienen als Produktionsstämme die Mikroorganismen der Spezies E.coli, die in Tabelle 1 aufgeführt sind und mit dem Hybridplasmid pCAR6 transformiert sind,sowie deren Deszendenten und Mutanten.

Die Mikroorganismen E.coli XL1-Blue (DSM-Nr. 6551) und E.coli DH5 (DMS-Nr. 7053) wurden, wie bereits beschrieben, hinterlegt.

Wird beispielsweise aus Tabelle 1 E.coli MC4100 (beschrieben in Mol.Gen.Genet. 192, 1983, S.293-294) als Wirtsstamm angewendet, erfolgt die Expression des stereoselektiven Hydrolase-Gens (rad) unter der Kontrolle des Promotors P$_{Lac}$ konstitutiv (permanent), aufgrund einer Deletion im lac-Operon (Lactose-Operon) (Deletion von (argF - lac) U169). Demnach wird das lac-Repressorgen lacI nicht gebildet (Repressorgen-negativer Mikroorganismus). Werden beispielsweise aus Tabelle 1 E.coli K12 (erhältlich unter der DSM-Nr. 498) oder E.coli HB101 (H.W.Boyer und D. Roulland-Dussoix, J.Mol.Bio. 41 (1969), S.459-472) als Wirtsstämme angewendet, wird die Expression des Hydrolase-Gens (rad) mit IPTG induziert, aufgrund der Anwesenheit des Repressorgens lacI (Repressorgen-positiver Mikroorganismus).

g) Biotransformation

Erfindungsgemäss können zur Biotransformation alle Mikroorganismen (Produktionsstämme) angewendet werden, die mit einem Gen, das eine stereospezifische Hydrolase bildet, transformiert sind und damit stereospezifisch das R-(-)-2,2-DMCPCA zur Säure hydrolysieren.

Zweckmässig wird die Biotransformation mit Mikroorganismen durchgeführt, die mit einem Hydrolase-Gen (rad), dessen Restriktionskarte in Fig. 1 charakterisiert ist, transformiert sind. Geeignet sind auch Mikroorganismen, die mit einem DNA-Fragment, das für ein Polypeptid mit stereospezifischer Hydrolase-Aktivität codiert, dessen Aminosäuresequenz in Fig. 3 dargestellt ist, transformiert sind. Ebenso geeignet sind Mikroorganismen, die mit einem DNA-Fragment das mit dem in Fig. 3 dargestelltem DNA-Fragment hybridisiert und das für ein Polypeptid mit stereospezifischer Hydrolase-Aktivität codiert, transformiert sind.

Für das Verfahren besonders geeignet sind, wie bereits beschrieben die Mikroorganismen der Spezies E.coli (Tabelle 1), transformiert mit dem Hybridplasmid pCAR 5 oder pCAR6, insbesondere die, die mit Hybridplasmid pCAR6 transformiert sind.

Ebenso geeignet sind die zellfreien Enzyme (die stereospezifischen Hydrolasen) aus diesen Mikroorganismen. Diese zellfreien Enzyme können durch fachmännisch übliches Aufschliessen der Mikroorganismenzellen gewonnen werden. Hierzu kann beispielsweise die Ultraschall-, French-Press- oder Lysozym-Methode angewendet werden. Diese zellfreien Enzyme können dann für die Durchführung des Verfahrens auf einem geeigneten Trägermaterial nach fachmännisch üblichen Methoden immobilisiert werden.

Vorzugsweise wird das Verfahren mit ruhenden Mikroorganismenzellen (nicht wachsende Zellen) durchgeführt, die zuvor entsprechend ihrem Expressionssystem induziert werden. Das heisst, werden Repressorgen-positive Mikroorganismen für das Verfahren eingesetzt,wie z.B. E.coli XL1-Blue oder E.coli DH5, die mit dem Hybridplasmid pCAR6 transformiert sind, erfolgt die Induktion mit IPTG. Werden für das Verfahren z.B. Repressorgen-negative (d.h. Fehlen des Repressorgens) Mikroorganismen der Spezies E.coli, wie z.B. E.coli MC4100, eingesetzt, erfolgt die Expression des Hydrolase-Gens (rad) permanent (konstitutiv).

In einer besonders bevorzugten Ausführungsform wird die spezifische Hydrolase-Aktivität der Mikroorganismen mit $C_1$-$C_4$-Alkoholen erhöht.

Als $C_1$-$C_4$-Alkohole können beispielsweise Methanol, Ethanol, Propanol, Isopropanol oder Butanol angewendet werden. Vorzugsweise wird Methanol oder Ethanol angewendet.

Als Medium für das Verfahren können die in der Fachwelt üblichen dienen, wie beispielsweise niedermolare Phosphat-Puffer, ein Mineralsalzmedium gemäss Kulla et al., Arch.Microbiol. 135, (1983), S.1-7, oder auch HEPES-Puffer (N-2-Hydroxyethylpiperazin-2'-ethansulfonsäure). Vorzugsweise wird das Verfahren in einem niedermolaren Phosphat-Puffer durchgeführt.

Zweckmässig enthält das Medium für die Biotransformation racemisches R,S-(±)2,2-DMCPCA in einer Menge von 0,2 bis 5 Gew.%, vorzugsweise 0,2 bis 2 Gew.%.

Zweckmässig wird die Biotransformation in einem Bereich von pH 6 bis 11, vorzugsweise in einem Bereich von pH 6,5 bis 10 durchgeführt.

Die Temperatur für die Biotransformation liegt zweckmässig zwischen 15 und 55°C, vorzugsweise zwischen 20 und 40°C

Nach einer übliche Umsetzungsdauer von 1 bis 30 h, vorzugsweise von 5 bis 25 h, ist das R-(-)-2,2-DMCPCA vollständig zur entsprechenden Säure umgewandelt, wobei optisch reines S-(+)-2,2-DMCPCA anfällt. Das so gewonnene S-(+)-2,2-DMCPCA kann dann beispielsweise durch Extraktion, Elektrodialyse oder durch Trocknung gewonnen werden.

Beispiel 1

1.1 Präparation chromosomaler DNA von Comamonas acidovorans A:18

Die chromosomale DNA einer frischen Übernachtkultur von Comamonas acidovorans A:18 (100 ml Nutrient Yeast Broth, 30°C) wurde nach der modifizierten Methode von R.H.Chesney et al. (J.Mol.Biol. 130 (1979), 161-173) isoliert: Die abzentrifugierten Zellen (15 min, 6'500 X g, 4°C) wurden in Tris-HCl-Puffer (2,25 ml, 0,05 mol/l), pH 8,0, 10% (w/v) Saccharose resuspendiert.

Nach Zugabe von 375 μl Lysozymlösung (10 mg/ml 0,25 mol/l Tris-HCl-Puffer, pH 8,0) und 900 μl 0,1 mol/l EDTA, pH 8,0, wurde die Suspension für 10 min auf Eis gekühlt.

Darauf folgte die Zugabe von 450 μl 5% (w/v) SDS und von 50 μl Ribonuklease (10 mg/ml $H_2O$) und eine Inkubation bei 37°C für 30 min. Die Inkubation wurde nach Zugabe einer Spatelspitze Proteinase K und 400 μl Pronase (20 ml/ml $H_2O$) für 2 h fortgesetzt.

Es wurde nach Mischen mit 4,3 g CsCl zentrifugiert (30 min, 40'000 X g, 20°C), mit 250 μl Ethidiumbromid (10 mg/ml) versetzt und in der Ultrazentrifuge (VTi 65.2-Röhrchen) zentrifugiert (mehr als 8 h, 246'000 X g, 20°C). Unter langwelligem UV-Licht wurde die DNA-Bande aus dem Röhrchen abgesaugt.

Nach Zugabe des 4-fachen Volumens TE-Puffer (10 mmol/l Tris-HCl, pH 8,0, 1 mmol/l EDTA) wurde das Ethidiumbromid dreimal mit Wasser gesättigtem n-Butanol extrahiert. Die DNA wurde mit Isopropanol präzipitiert, in TE-Puffer aufgenommen und 15 min bei 65°C inkubiert. Das Präparat konnte bei 4°C aufbewahrt werden.

## 1.2 Restriktion und Ligation der chromosomalen DNA

5 µg Comamonas acidovorans A:18 (DSM-Nr. 6315)-DNA und 4,5 µg Vektor-DNA (pBLUESCRIPT-KS+[R]) wurden jeweils mit 20 Units Restriktionsenzym EcoRI in einem Totalvolumen Restriktionspuffer von 100 µl geschnitten (6,5 h bei 37°C). Die DNAs wurden mit Ethanol präzipitiert und im Speed Vac [R] Concentrator getrocknet. Die Niederschläge wurden im Ligationspuffer (20 mmol/l Tris-Puffer, 10 mmol/l DTT (Dithiothreitol), 10 mmol/l $MgCl_2$, 0,6 mol/l ATP (Adenosintriphosphat, pH 7,2) aufgenommen und vereinigt (Ligationsvolumen 100 µl).

Nach Zugabe von 1 Unit T4-DNA-Ligase wurde über Nacht bei 13°C inkubiert.

Die DNA des Ligationsgemisches wurde mit Isopropanol präzipitiert und in 30 µl Wasser zur Transformation aufgenommen.

## 1.3 Transformation von E.coli XL1-Blue[R] und Selektion

Kompetente Zellen von E.coli XL1-Blue[R] wurden mittels Elektroporation mit dem Ligationsgemisch nach der beschriebenen Methode von S.Fiedler und R.Wirth (Analyt.Biochem. 170 (1988), 38-44) transformiert.

Zum Plasmidnachweis wurde auf Nutrient Agar mit Ampicillin (100 µg/ml) und zum "Insert"-Nachweis mit 0,5 mmol/l IPTG (Isopropyl-β-D-Thiogalaktosid) und X-Gal (30 µg/ml, 5-Brom-4-Chlor-3-Indolyl-β-D-galaktopyranosid) bei Inkubation bei 37°C selektioniert.

Bei einer Transformationsfrequenz von 1,7 X $10^8$ cfu/ml ("colony forming units" ≙ lebenden Zellen) besassen nahezu alle Klone ein EcoRI-"Insert".

## Beispiel 2

## 2. Screening der Comamonas acidovorans A:18-Genbank nach dem R-spezifischen Amidase-Gen

Klone mit Hybridplasmiden (EcoRI-"Insert") wurden auf Minimalmedium-Agar nach H.Kulla et al. (Arch.Microbiol. 135 (1983), 1-7) mit 0,2% (v/v) Glycerin als C-Quelle, 0,15% (w/v) R,S-(±)-2,2-DMCPCA als einzige N-Quelle und 0,5 mmol/l IPTG als Induktor des lac-Promotors, sowie Ampicillin (5 µg/ml) zur Plasmidstabilisierung, auf ihre Wachstumsfähigkeit überprüft. Nur Klone, welche das intakte Hydrolase-Gen rad auf dem DNA-"Insert" im Plasmid enthalten, waren fähig das R-(-)-2,2-DMCPCA als N-Quelle zu verwerten, dieses in die gesuchte R-Säure umzusetzen und auf diesem Minimalmedium zu wachsen.

Solchermassen selektionierte Klone enthielten alle ein Hybridplasmid aus Vektor pBLUESCRIPT-KS+[R] mit einem EcoRI-"Insert" von ca. 23 kb.

Das Plasmid pCAR1 wurde isoliert und näher charakterisiert.

## Beispiel 3

## 3.1 Isolation der R-spezifischen Hydrolase aus Comamonas acidovorans A:18 und N-terminale Peptidanalyse

## a) Präparation von zellfreiem Extrakt

16 l einer Zellsuspension von Comamonas acidovorans A:18 (DSM-Nr. 6315) mit einer Hydrolase-Aktivität bei 37°C von 0,6 g R-(-)-2,2 DMCPCS/l/h/optische Dichte bei 650 nm ($OD_{650}$) = 1, die zuvor mit R,S-(±)-DMCPCA induziert wurden, wurden auf 700 ml ($OD_{650}$ = 33,5) konzentriert. Anschliessend wurden die Zellen mehrmals zentrifugiert, im HEPES-Puffer resuspendiert und danach im HEPES-Puffer (40 ml) aufgenommen, wobei das Volumen der gesamten Zellsuspension dann 95 ml ($OD_{650}$ = 210) betrug. Die Hydrolase-Aktivität wurde bei 30°C bestimmt und betrug 0,34 g Produkt/l/h/$OD_{650}$ = 1. Anschliessend wurden die Zellen 2 mal in der French-press bei einem Druck von 1200 bar aufgeschlossen. Um ein zellfreies Extrakt zu erhalten wurde diese Suspension bei 20000 X g 20 min lang zentrifugiert.

Es wurden 50 ml Extrakt mit einer Proteinmenge (gemessen nach der Bradford-Methode) von 39,3 mg/ml und mit einer Hydrolase-Aktivität bei 30°C von 12,5 g R-(-)-2,2-DMCPCS/l/h/mg Protein erhalten.

b) Chromatographie

Dieses Roh-Zell-Extrakt (50 ml) wurde auf eine mit Q-Sepharose (Pharmacia) gefüllte Säule, welche gegen einen HEPES-Puffer (0,1 mol/l, pH 7,5) äquilibriert worden war, aufgetragen.

Diese Säule wurde noch 2 mal mit dem selben Puffer durchspült und dann wurden die Proteine mit einem HEPES-Puffer-Gradienten (0,1-1 mol/l) eluiert. Insgesamt wurden mit HEPES-Puffer (1 mol/l) 140 ml Proteinlösung mit Hydrolase-Aktivität eluiert, die dann mittels Ultrafiltration (Amicon-Membran YM10) konzentriert wurden. Die Proteinmenge dieser Enzymlösung betrug 131 mg/ml wobei die Hydrolase-Aktivität 1 μmol/min/ng Protein betrug.

Anschliessend wurden 2 ml dieser Proteinlösung auf eine mit Superose-12 (Pharmacia) gefüllte Säule, die gegen HEPES-Puffer (0,1 mol/l; pH 7,5) äquilibriert worden war, aufgetragen. Insgesamt wurden mit diesem Puffer 36 ml Proteinlösung eluiert. Diese wurde ebenso durch Ultrafiltration (Amicon-Membran YM10) konzentriert. Die Proteinmenge betrug 20,1 mg/ml wobei die Hydrolase-Aktivität 1,2 μmol/min/ng Protein betrug.

Die so erhaltene Proteinlösung wurde dann auf eine mit dem Anionen-Austauscher Li Chirospher 2000 TMAE (Trimethylammoniumethylsalz) (Merck) gefüllte Säule, die gegen HEPES-Puffer (0,1 mol/l; pH 7,5) äquilibriert worden war, aufgetragen.

Nach Durchspülen der Säule mit dem gleichen Puffer wurde die Proteinlösung mit einem NaCl-Gradienten (0-1 mol/l) im gleichen Puffer eluiert. Die Proteinkonzentration betrug 15 mg/ml, wobei die Hydrolaseaktivität 1,2 μmol/min/ng Protein betrug.

c) Identifikation der Hydrolase mittels 1- und 2-dimensionaler Elektrophorese

Im Roh-Zell-Extrakt wurde das Hydrolase-Protein mittels SDS-PAGE identifiziert. Dabei wurde nicht-induziertes mit induziertem Zell-Extrakt auf dem SDS-PAGE verglichen (Induktion mit R,S-(±)-2,2-DMCPCA).

Im induzierten Zell-Extrakt wurde eine Proteinbande mit einem Molekulargewicht um 46000 nachgewiesen. Die mittels Chromatographie erhaltenen Proteinfraktionen, mit Hydrolase-Aktivität, wurden ebenfalls mittels SDS-PAGE analysiert. Das Protein mit einem Molekulargewicht um 46000 wurde durch diese chromatographische Reinigung angereichert, wobei es nach der dritten Chromatographie zu ca 80% angereichert war. Diese zu 80% reine Probe wurde dann mittels bidimensionaler Elektrophorese (2-D SDS-PAGE) analysiert. Durch diese Methode konnte ein Protein-"Spot" mit einem Molekulargewicht um 46000, welches dem der Hydrolase entsprach, nachgewiesen werden.

d) Sequenzierung

Der mittels 2-D SDS-PAGE erhaltene Protein-"Spot" wurde dann auf eine PVDF (Polyvinylidendifluorid)-Membran geblottet und aus der Membran herausgeschnitten. Anschliessend wurde dieses Protein direkt nach der Methode von Hochstrasser et al. (Applied and Theoretical Electrophoresis 1, S.73-76, 1988, "HDL particle-associated proteins in plasma and cerebrospinal fluid") sequenziert.

Nach dieser Methode wurden 21 Aminosäuren (AS) der N-terminalen Aminosäuresequenz, identifiziert.

Beispiel 4

4. Lokalisation des Hydrolase-Gens (rad) auf dem klonierten EcoRI-Fragment

4.1 Grobe Restriktionskarte von pCAR1

Durch Restriktionsanalyse nach herkömmlichem Vorgehen (Current Protocols Molecular Biology, John Wiley and Sons, New York, 1987, Abschnitt 2, wurde eine grobe Restriktionskarte vom pCAR1 bezüglich EcoRV, PvuII, KspI, SmaI, PstI, StuI, BamHI erstellt.

4.2 Formulierung von gemischten DNA-Oligomeren beruhend auf der N-terminalen Peptidsequenz der Hydrolase

Aufgrund des genetischen Codes konnten zwei gemischte DNA-Oligomere für die N-terminale Peptidsequenz der Comamonas acidovorans A:18 Hydrolase formuliert und mit einer DNA-Synthese-Maschine synthetisiert werden:

## DNA-Oligomer (Gemisch)

```
                 T     T   TCT   A   T T     T
     5'  ATG  AAC  GAC  AGC  GAG  CTC  CAC  CA  3'
                             C            C
                             A            A


     AS  Met  Asn  Asp  Ser  Glu  Leu  His       AS
```

## DNA-"Antisense" Oligomer (Gemisch)

```
              A     C    T    T    T    T
     5'  TG  GAT  TTC  CCG  CCC  CAC  CTC  3'
                       G    G    G
                       A    A


     AS       Ile  Glu  Arg  Gly  Val  Glu  AS
```

### 4.3 "Southern Blot-Hybridisierung" von Restriktionsfragmenten des Plasmids pCAR1

Die über Agarosegel-Elektrophorese (0,6%) aufgetrennten DNA-Fragmente, die nach unterschiedlichen Restriktionen (BamHI, PstI, EcoRI) von pCAR1 erhalten wurden, wurden über das bekannte "Southern Blot-Verfahren" auf Nitrocellulose übertragen. (Current Protocols in Molecular Biology, John Wiley and Sons, New York, 1987, Abschnitt 2.9ff).

Die DNA-Oligomere wurden gleichermassen mit [$^{32}$P]-Gamma-ATP endmarkiert:

400 ng DNA-Oligomere, 22 $\mu$Ci $^{32}$P-Gamma-ATP, 11 Units Polynukleotidkinase phosphatfrei, in total 25 $\mu$l Polynukleotidkinase-Puffer (0,05 mol/l Tris-HCl, pH 7,5, 0,01 mol/l MgCl$_2$, 5 mmol/l DTT) wurden für 30 min bei 37°C inkubiert. Danach Aufreinigung durch Sephadex G-25-Gelfiltration (Pharmacia) und Hybridisierung gegen die "Southern Blots" nach dem bekannten Vorgehen (in der oben genannten Literaturstelle).

Durch Hybridisierung gegen die der N-terminalen Proteinsequenz entsprechenden Nukleotid-Oligomeren konnte ein 2,3 kb grosses EcoRI-BamHI-DNA-Fragment oder ein 1,85 kb grosses PvuII-BamHI-DNA-Fragment auf dem Hybridplasmid pCAR1 markiert werden.

### 4.4 Subklonierungen des Hydrolase-Gens (rad)

Das 2,3 kb grosse EcoRI-BamHI-DNA Fragment, oder das 1,85 kb grosse PvuII-BamHI-DNA-Fragment, welches für die R-spezifische Hydrolase aus Comamonas acidovorans A:18 codiert, wurde in gleichermassen verdaute Vektor-DNA pBLUESCRIPT-KS+$^R$ oder pBLUESCRIPT SK+$^R$ inseriert.

Nur eine Orientierung des "Inserts" zum Promotor P$_{Lac}$ zeigte in den Klonen nach IPTG-Induktion die gesuchte Hydrolase Aktivität.

Der Vektor pBLUESCRIPT-KS+$^R$ mit dem 2,3 kb grossen EcoRI-BamHI-DNA-Fragment wurde als Hybridplasmid pCAR6 bezeichnet. Der Vektor pBLUESCRIPT-KS+$^R$ mit dem 1,85 kb grossen PvuII-BamHI-DNA-Fragment wurde als Hybridplasmid pCAR5 bezeichnet.

### Beispiel 5

### 5. Aktivitätsbestimmung der R-(-)-2,2-DMCPCA-Hydrolase

Zur Aktivitätsbestimmung der Hydrolase wurde die Mikroorganismensuspension auf eine optische Dichte von 0,5 bei 650 nm eingestellt. Als Medium diente ein Phosphat-Puffer (10 mmol/l), pH 7,0, mit 0,2 Gew.% R,S-($\pm$)-2,2-DMCPCA. Diese Suspension wurde 4 h bei 30°C unter Schütteln inkubiert. Das durch die Hydrolase freigesetzte NH$_4^+$ oder die R-(-)-2,2-DMCPCS wurde gemessen und die Aktivität als g R-(-)-2,2-DMCPCA umgesetzt pro l/h/optische Dichte bei 65C nm ausgedrückt, vorausgesetzt, dass 1 mmol gebildetes NH$_4^+$ = 1 mmol umgesetztem R-(-)-2,2-DMCPCA entspricht.

Beispiel 6

Herstellung von S-(+)-2,2-DMCPCA

E.coli K12 mit Hybridplasmid pCAR6 zeigte im Mineralsalzmedium enthaltend 0,2% (v/v) Glycerin und 0,15 Gew. % R,S-(±)-2,2-DMCPCA eine spezifische Hydrolase-Aktivität von 1,2 g R-(-)-2,2-DMCPCS/l/h/$OD_{650}$ nach IPTG-Induktion. Die Umsetzung von R-(-)-2,2-DMCPCA zur R-(-)-Säure wurde über $NH_4^+$ -Freisetzung und GC-Analyse bestätigt. Das Zielprodukt S-(+)-2,2-DMCPCA blieb im racemischen Gemisch unverändert.

Entsprechend zu E.coli K12 wurden die in Tabelle 1 aufgeführten Mikroorganismen kultiviert und die Ergebnisse der Umsetzung in Tabelle 1 dargestellt.

Tabelle 1

Stereospezifische Hydrolase-Aktivität in verschiedenen E.coli-Stämmen

| Stamm | spez. Aktivität g/l/h/OD | Faktor | Stabilität in % [4] | Max. OD $_{650}$ nm | Gesamt-Aktivit. g/l/h |
|---|---|---|---|---|---|
| Comamonas acidovorans (nicht erfindungsgem.) A:18 [1] | 0,5 | 1 | - | 6 | 3,0 |
| E.coli K12/pCAR6 [2] | 1,2 | 2,4 | 90 | nt | - |
| E.coli HB101/pCAR6 [2] | 0,25 | 0,5 | nt | nt | - |
| E.coli MC4100/pCAR6 [3] | 0,53 | 1 | 89 | nt | - |
| E.coli XL1BLUE/pCAR6 [5] (DSM-Nr. 6551) | 0,5 | 1 | 90 | 30 | 15,0 |
| E.coli DH5/pCAR6 [5] (DSM-Nr. 7053) | 2,1 | 4,2 | 100 | 30 | 63,0 |

1) Induktion mit Amid, 2) Induktion mit IPTG, 3) Konstitutiv, 4) Plasmidstabilität nach 24 h, ohne Antibiotikaselektion, nt = nicht getestet, 5) ohne Induktion

Beispiel 7

Aktivitätstest mit $C_1$-$C_4$-Alkoholen

Die Aktivitätstests wurden zunächst mit <u>Comamonas acidovorans</u> A:18 bei 37°C mit 0,5% R,S-2,2-DMCPCA in 10 mM Kaliumphosphat-Puffer bei pH 7,0 durchgeführt. Kontrolle ohne Lösungsmittel, Testversuche mit 5 bis 16 Vol% Lösungsmittel. Die Berechnung der spezifischen Aktivität erfolgte wie in Beispiel 5 beschrieben.

| Lösungsmittel (Vol.%) | Aktivität für die Umsetzung von g R-2,2-DMCPCA/l/h/OD$_{650}$ nm |
|---|---|
| --- | 0,64 |
| Ethanol (10) | 1,24 |
| Isopropanol (10) | 1,85 |
| Methanol (5) | 1,79 |
| Methanol (10) | 1,54 |
| Methanol (16) | 1,66 |

Bei Biotransformationen im 20 l-Fermenter mit 2 bis 3% R,S-2,2-DMCPCA (37°C, 10 mM, Kaliumphosphatpuffer, pH 7,0) konnte mit der Zugabe von 5 - 7,5 Vol% Methanol oder Ethanol eine Verkürzung der Umsetzungsdauer und eine höhere Ausbeute an S-(+)-2,2-DMCPCA (Selektivitätserhöhung) erreicht werden. Der gleiche Effekt wurde bei dem <u>E.coli</u>-Stamm XL1-Blue mit dem Hydrolase-Gen beobachtet. Die Ergebnisse sind in Tabelle 2 zusammengefasst.

Tabelle 2

| (Es wurden von jedem Stamm gleiche Aktivitäten (in Wasser) eingesetzt) | | | | | |
|---|---|---|---|---|---|
| Stamm | R,S-2,2-DMCPCA (%) | Lösungsmittel (Vol.%) | Dauer (h) | ee (%) | Ausbeute (%) *) |
| A:18 | 2,0 | --- | 22 | 99 | 41,5 |
| A:18 | 2,3 | Methanol (7,5) | 15 | 99,2 | 47 |
| XL1/pCAR6 | 2,8 | --- | 24 | 100 | 36 |
| XL1/pCAR6 | 2,8 | Methanol (7,5) | 7 | 98,2 | 46 |
| XL1/pCAR6 | 2,8 | Ethanol (5) | 7 | 98,6 | 44 |

*) an S-(+)-2,2-DMCPCA bezogen auf eingesetztes R,S-DMCPCA ee: Enantiomeren-Reinheit

Beispiel 8

Immobilisierung der stereospezifischen Hydrolase von E.coli XL1-Blue/pCAR6

Das zellfreie Extrakt (288 ml) von <u>E.coli</u> XL1-Blue/pCAR6 enthaltend 28 mg Protein/ml mit einer Hydrolase-Aktivität bei 37°C von 0,29 µmol R-(-)-2,2-DMCPCA/min · mg Protein wurde zunächst durch Säulenchromatographie an Q-Sepharose (Pharmacia) vorgereinigt. Hierzu wurde das Hydrolase-Protein mit einem NaCl-Gradienten (0-1 mol/l) in einem Tris-HCl-Puffer (0,1 molar, pH 7,5) eluiert. Das Protein mit Hydrolase-Aktivität welches zwischen 40% und 80% des NaCl-Gradienten eluiert worden war, wurde dann durch Ultrafiltration (Amicon Membran YM10) konzentriert und mittels Gelfiltration (PD-10, Sephadex G-25M, Pharmacia LKB) entsalzt. Das Endvolumen betrug dann 47 ml in Kaliumphosphat-Puffer (0,1 molar, pH 7,0) enthaltend 67 mg Protein/ml mit einer Hydrolase-Aktivität bei 37°C von 0,69 µmol R-(-)-2,2-DMCPCA/min · mg Protein. Anschliessend wurde diese vorgereinigte stereospezifische Hydrolase auf Eupergit C als Trägermaterial (Röhm Pharma GmbH, Weiterstadt, BRD) immobilisiert. Hierzu wurden die Oxirangruppen des unlöslichen Trägermaterials kovalent an die freien Aminogruppen des Hydrolase-Proteins gebunden. Die Immobilisierung wurde 90 h lang bei Raumtemperatur in Kaliumphosphat-Puffer (1 molar, pH 8,5) durchgeführt. Es wurden 10,2 mg immobilisiertes Protein/g Feuchtgewicht Eupergit C, mit einer Hydrolase-Aktivität bei 37°C von 1,5 µmol R-(-)2,2-DMCPCA/min · g Feuchtgewicht Eupergit C, erhalten. Die Stabilität der immobilisierten Hydrolase bei 37°C im Kaliumphosphat-Puffer (10 mmolar, pH 8,5) enthaltend 0,5 Gew.% R,S-(±)-2,2-DMCPCA ist in Tabelle 3 dargestellt.

Tabelle 3

| Dauer h | Aktivität der immobilisierten Hydrolase (µmol R-(-)-2,2-DMCPCA/min · g Feuchtgewicht Eupergit C) |
|---|---|
| 0 - 90 | 1,5 |

Tabelle 3   (fortgesetzt)

| Dauer h | Aktivität der immobilisierten Hydrolase (µmol R-(-)-2,2-DMCPCA/min · g Feuchtgewicht Eupergit C) |
|---|---|
| 90 - 185 | 0,68 |

**Patentansprüche**

1.  Gentechnologisches Verfahren zur Herstellung von S-(+)-2,2-Dimethylcyclopropancarboxamid, dadurch gekennzeichnet, daß man im racemischen R,S-(±)-2,2-Dimethylcyclopropancarboxamid das R-(-)-2,2-Dimethylcyclopropancarboxamid mittels Mikroorganismen, die mit einem für eine stereospezifische Hydrolase codierenden Gen transformiert sind, zur R-(-)-2,2-Dimethylcyclopropancarbonsäure biotransformiert, wobei optisch aktives S-(+)-2,2-Dimethylcyclopropancarboxamid anfällt, das gegebenenfalls isoliert wird.

2.  Verfahren nach Patentanspruch 1, dadurch gekennzeichnet, dass man die Biotransformation mit Mikroorganismen durchführt, die mit einem Gen für eine stereospezifische Hydrolase, das durch nachstehende Restriktionskarte (Fig. 1) charakterisiert ist, transformiert sind;

```
                                ──  EcoRI   -0,45

                                     EcoRV   -0,43



                                ──  PvuII    0




                    ┌──                        0,29
                    │
                    │           ──  KspI     0,50
                    │           ──  SmaI     0,55
                    │           ──  PstI     0,66
                    │           ──  SmaI     0,74
                 ┌─┐
                 │H│
                 │a│
                 │d│
                 └─┘
                    │
                    │           ──  StuI     1,28
                    │
                    │               SmaI     1,29
                    └──
                                               1,57



                                ──  PstI     1,81
                                    BamHI    1,85
```

3.  Verfahren nach einem der Patentansprüche 1 und 2, dadurch gekennzeichnet, dass man die Biotransformation mit Mikroorganismen durchführt, die mit einem DNA-Fragment, codierend für ein Polypeptid mit stereospezifischer Hydrolase-Aktivität, dessen Aminosäuresequenz in Fig. 3 dargestellt ist, transformiert sind.

4.  Verfahren nach mindestens einem der Patentansprüche 1 bis 3, dadurch gekennzeichnet, dass man die Biotransformation mit Mikroorganismen durchführt, die mit einem DNA-Fragment, das mit dem in Fig. 3 dargestellten DNA-Fragment hybridisiert und das für ein Polypeptid mit stereospezifischer Hydrolase-Aktivität codiert, transformiert sind.

5.  Verfahren nach mindestens einem der Patentansprüche 1 bis 4, dadurch gekennzeichnet, dass man die Biotransformation mit Mikroorganismen der Gattung Escherichia, Pseudomonas, Comamonas, Acinetobacter, Rhizobium

oder <u>Agrobacterium</u> durchführt.

6. Verfahren nach mindestens einem der Patentansprüche 1 bis 5, dadurch gekennzeichnet, dass man die Biotransformation mit Mikroorganismen der Spezies <u>Escherichia</u> <u>coli</u> durchführt.

7. Verfahren nach mindestens einem der Patentansprüche 1 bis 6, dadurch gekennzeichnet, dass man die Biotransformation mit Mikroorganismen der Spezies <u>Escherichia</u> <u>coli</u> XL1-Blue (DSM-Nr. 6551) durchführt, die mit dem Hybridplasmid pCAR6 transformiert sind.

8. Verfahren nach mindestens einem der Patentansprüche 1 bis 6, dadurch gekennzeichnet, dass man die Biotransformation mit Mikroorganismen der Spezies <u>Escherichia</u> <u>coli</u> DH5 (DSM-Nr. 7053) durchführt, die mit dem Hybridplasmid pCAR6 transformiert sind.

9. Gentechnologisches Verfahren zur Herstellung von S-(+)-2,2-Dimethylcyclopropancarboxamid, dadurch gekennzeichnet, daß man im racemischen R,S-(±)-2,2-Dimethylcyclopropancarboxamid das R-(-)-2,2-Dimethylcyclopropancarboxamid mit einer immobilisierten stereospezifischen Hydrolase, erhalten aus einem mit einem für diese stereospezifische Hydrolase codierenden Gen transformierten Mikroorganismus, zur R-(-)-2,2-Dimethylcyclopropancarbonsäure biotransformiert, wobei optisch aktives S-(+)-2,2-Dimethylcyclopropancarboxamid anfällt, das gegebenenfalls isoliert wird.

10. Verfahren nach mindestens einem der Patenansprüche 1 bis 9, dadurch gekennzeichnet, dass man die Biotransformation in einem Medium, enthaltend racemisches R,S-(±)-2,2-Dimethylcyclopropancarboxamid in einer Menge von 0,2 bis 5 Gew.%, durchführt.

11. Verfahren nach mindestens einem der Patentansprüche 1 bis 10, dadurch gekennzeichnet, dass man die Biotransformation bei einem pH von 6 bis 11 und bei einer Temperatur von 15 bis 55°C durchführt.

12. Eine DNA codierend für eine stereospezifische Hydrolase, charakterisiert durch nachstehende Restriktionskarte (Fig. 1):

EcoRI -0,45
EcoRV -0,43

PvuII 0

0,29

KspI 0,50
SmaI 0,55
PstI 0,66
SmaI 0,74

rad

StuI 1,28
SmaI 1,29

1,57

PstI 1,81
BamHI 1,85

13. Ein DNA-Fragment codierend für ein Polypeptid mit stereospezifischer Hydrolase-Aktivität, dessen Aminosäuresequenz in Fig. 3 dargestellt ist.

14. Ein DNA-Fragment, das mit dem in Fig. 3 dargestellten DNA-Fragment hybridisiert und das für ein Polypeptid mit stereospezifischer Hydrolase-Aktivität codiert.

15. Eine DNA oder ein DNA-Fragment nach Patentanspruch 12, 13 oder 14 in Hybridplasmid pCAR6, hinterlegt in Escherichia coli XL1-Blue (DSM-Nr. 6551).

**16.** Eine DNA oder ein DNA-Fragment nach Patentanspruch 12, 13 oder 14 in Hybridplasmid pCAR6, hinterlegt in <u>Escherichia coli</u> DH5 (DSM-Nr. 7053).

**17.** Hybridplasmid, bestehend aus einem Expressionsvektor mit der darin eingefügten DNA oder dem DNA-Fragment nach Patentanspruch 12, 13 oder 14.

**18.** Hybridplasmid pCAR6 bestehend aus der DNA oder dem DNA-Fragment gemäss Patentanspruch 12, 13 oder 14 und Expressionsvektor pBLUESCRIPT-KS+, hinterlegt in <u>Escherichia coli</u> XL1-Blue (DSM-Nr. 6551).

**19.** Hybridplasmid pCAR6 bestehend aus der DNA oder dem DNA-Fragment gemäss Patentanspruch 12, 13 oder 14 und Expressionsvektor pBLUESCRIPT-KS+, hinterlegt in <u>Escherichia coli</u> DH5 (DSM-Nr. 7053).

**20.** Mikroorganismen, die mit einem Hybridplasmid nach Patentanspruch 17, 18 oder 19 transformiert sind.

**21.** Mikroorganismen nach Patentanspruch 20 der Spezies <u>Escherichia coli</u> XL1-Blue (DSM-Nr. 6551), die mit dem Hybridplasmid pCAR6 transformiert sind.

**22.** Mikroorganismen nach Patentanspruch 20 der Spezies <u>Escherichia coli</u> DH5 (DSM-Nr. 7053), die mit dem Hybridplasmid pCAR6 transformiert sind.


**Claims**

1. A genetic engineering method for producing S-(+)-2,2-dimethylcyclopropane carboxamide, characterised in that the R-(-)-2,2-dimethylcyclopropane carboxamide in the racemic R,S-(±)-2,2-dimethylcyclopropane carboxamide is bio-transformed into R-(-)-2,2-dimethylcyclopropane carboxylic acid by means of micro-organisms transformed by a gene encoding for a stereospecific hydrolase, involving the production of optically active S-(+)-2,2-dimethylcyclopropane carboxamide which is isolated in a given case.

2. The method according to claim 1, characterised in that bio-transformation is performed by means of micro; organisms transformed by a gene for a stereospecific hydrolase which is characterised by the restriction map (Fig. 1) shown below:

```
                                    EcoRI  -0.45
                                    EcoRV  -0.43



                                    PvuII   0



                                            0.29


                                    KspI    0.50
                                    SmaI    0.55
                                    PstI    0.66
                                    SmaI    0.74

              r
              a
              d


                                    StuI    1.28

                                    SmaI    1.29


                                            1.57



                                    PstI    1.81
                                    BamHI   1.85
```

3. The method according to one of claims 1 and 2, characterised in that bio-transformation is performed by means of micro-organisms transformed by a DNA fragment encoding for a polypeptide having stereospecific hydrolase activity, the amino-acid sequence of which is represented in Fig. 3.

4. The method according to at least one of claims 1 to 3, characterised in that bio-transformation is performed by means of micro-organisms transformed by a DNA fragment which hybridises with the DNA fragment represented in Fig. 3 and which encodes for a polypeptide having stereospecific hydrolase activity.

5. The method according to at least one of claims 1 to 4, characterised in that bio-transformation is performed by means of micro-organisms of the genus <u>Escherichia, Pseudomonas, Comamonas, Acinetobacter, Rhizobium</u> or <u>Agrobacterium.</u>

6. The method according to at least one of claims 1 to 5, characterised in that bio-transformation is performed by means of micro-organisms of the species Escherichia coli.

7. The method according to at least one of claims 1 to 6, characterised in that bio-transformation is performed by means of micro-organisms of the species Escherichia coli XL1-Blue (DSM No. 6551) transformed by the hybrid plasmid pCAR6.

8. The method according to at least one of claims 1 to 6, characterised in that bio-transformation is performed by means of micro-organisms of the species Escherichia coli DH5 (DSM No. 7053) transformed by the hybrid plasmid pCAR6.

9. A genetic engineering method for producing S-(+)-2,2-dimethylcyclopropane carboxamide, characterised in that the R-(-)-2,2-dimethylcyclopropane carboxamide in racemic R,S-($\pm$)-2,2-dimethylcyclopropane carboxamide is bio-transformed into R-(-)-2,2-dimethylcyclopropane carboxylic acid by means of an immobilised stereospecific hydrolase obtained from a micro-organism transformed by a gene which encodes for this stereospecific hydrolase, involving the production of optically active S-(+)-2,2-dimethylcyclopropane carboxamide which is isolated in a given case.

10. The method according to at least one of claims 1 to 9, characterised in that bio-transformation is performed in a medium containing racemic R,S-($\pm$)-2,2-dimethylcyclopropane carboxamide in an amount of 0.2 to 5% (wt.).

11. The method according to at least one of claims 1 to 10, characterised in that bio-transformation is performed at a pH of 6 to 11 at a temperature of 15 to 55°C.

12. A DNA encoding for a stereospecific hydrolase, characterised by the restriction map (Fig. 1) shown below:

EcoRI -0.45
EcoRV -0.43
PvuII 0
0.29
KspI 0.50
SmaI 0.55
PstI 0.66
SmaI 0.74
StuI 1.28
SmaI 1.29
1.57
PstI 1.81
BamHI 1.85

13. A DNA fragment encoding for a polypeptide having stereospecific hydrolase activity, the amino-acid sequence of which is represented in Fig. 3.

14. A DNA fragment which hybridises with the DNA fragment represented in Fig. 3 and which encodes for a polypeptide having stereospecific hydrolase activity.

15. A DNA or a DNA fragment according to claim 12, 13 or 14 in hybrid plasmid pCAR6, deposited in Escherichia coli XL1-Blue (DSM No. 6551).

16. A DNA or a DNA fragment according to claim 12, 13 or 14 in hybrid plasmid pCAR6, deposited in Escherichia coli DH5 (DSM No. 7053).

**17.** A hybrid plasmid, consisting of an expression vector including the DNA or DNA fragment according to claim 12, 13 or 14 inserted therein.

**18.** Hybrid plasmid pCAR6, consisting of the DNA or the DNA fragment according to claim 12, 13 or 14 and expression vector pBLUESCRIPT-KS+, deposited in <u>Escherichia coli</u> XL1-Blue (DSM No. 6551).

**19.** Hybrid plasmid pCAR6, consisting of the DNA or the DNA fragment according to claim 12, 13 or 14 and expression vector pBLUESCRIPT-KS+, deposited in <u>Escherichia coli</u> DH5 (DSM No. 7053).

**20.** Micro-organisms transformed by a hybrid plasmid according to claim 17, 18 or 19

**21.** Micro-organisms according to claim 20 of the species <u>Escherichia coli</u> XL1-Blue (DSM No. 6551), transformed by the hybrid plasmid pCAR6

**22.** Micro-organisms according to claim 20 of the species <u>Escherichia coli</u> DH5 (DSM No. 7053), transformed by the hybrid plasmid pCAR6

**Revendications**

**1.** Procédé de génie génétique pour la préparation du S-(+)-2,2-diméthylcyclopropanecarboxamide, caractérisé en ce que, dans le R,S-(±)-2,2-diméthylcyclopropanecarboxamide racémique, on biotrans forme en l'acide R-(-)-2,2-diméthylcyclopropanecarboxylique le R-(-)-2,2-diméthylcyclopropanecarboxamide à l'aide de micro-organismes qui sont transformés avec un gène codant une hydrolase stéréospécifique, avec formation de S-(+)-2,2-diméthylcyclopropanecarboxamide optiquement actif qui est éventuellement isolé.

**2.** Procédé selon la revendication 1 caractérisé en ce que l'on réalise la biotransformation avec des micro-organismes qui sont transformés avec un gène d'une hydrolase stéréospécifique qui est caractérisé par la carte de restriction suivante (fig. 1):

EcoRI  -0,45
EcoRV  -0,43
PvuII   0
                0,29
KspI    0,50
SmaI    0,55
PstI    0,66
SmaI    0,74

StuI    1,28
SmaI    1,29
                1,57
PstI    1,81
BamHI   1,85

3. Procédé selon l'une des revendications 1 et 2 caractérisé en ce que l'on réalise la biotransformation avec des micro-organismes qui sont transformés avec un fragment d'ADN codant un polypeptide à activité d'hydrolase stéréospécifique dont la séquence d'acides aminés est représentée sur la figure 3.

4. Procédé selon au moins l'une des revendications 1 à 3 caractérisé en ce que l'on réalise la biotransformation avec des micro-organismes qui sont transformés avec un fragment d'ADN qui s'hybride avec le fragment d'ADN représenté sur la figure 3 et qui code un polypeptide à activité d'hydrolase stéréospécifique.

5. Procédé selon au moins l'une des revendications 1 à 4 caractérisé en ce que l'on réalise la biotransformation avec des micro-organismes du genre Escherichia, Pseudomonas, Comamonas, Acinetobacter, Rhizobium ou Agrobacterium.

EP 0 524 604 B1

6. Procédé selon au moins l'une des revendications 1 à 5 caractérisé en ce que l'on réalise la biotransformation avec des micro-organismes de l'espèce <u>Escherichia</u> <u>coli.</u>

7. Procédé selon au moins l'une des revendications 1 à 6 caractérisé en ce que l'on réalise la biotransformation avec des micro-organismes de l'espèce <u>Escherichia</u> <u>coli</u> XL1-Blue (n° DSM 6551) qui sont transformés avec le plasmide hybride pCAR6.

8. Procédé selon au moins l'une des revendications 1 à 6 caractérisé en ce que l'on réalise la biotransformation avec des micro-organismes de l'espèce <u>Escherichia</u> <u>coli</u> DH5 (n° DSM 7053) qui sont transformés avec le plasmide hybride pCAR6.

9. Procédé de génie génétique pour la préparation du S-(+)-2,2-diméthylcyclopropanecarboxamide, caractérisé en ce que, dans le R,S-(±)-2,2-diméthylcyclopropanecarboxamide racémique, on biotrans forme en l'acide R-(-)-2,2-diméthylcyclopropanecarboxylique le R-(-)-2,2-diméthylcyclopropanecarboxamide avec une hydrolase stéréospécifique immobilisée obtenue à partir d'un micro-organisme transformé avec un gène codant cette hydrolase stéréospécifique, avec formation de S-(+)-2,2-diméthylcyclopropanecarboxamide optiquement actif qui est éventuellement isolé.

10. Procédé selon au moins l'une des revendications 1 à 9 caractérisé en ce que l'on réalise la biotransformation dans un milieu contenant du R,S-(±)-2,2-diméthylcyclopropanecarboxamide racémique en une quantité de 0,2 à 5% en masse.

11. Procédé selon au moins l'une des revendications 1 à 10 caractérisé en ce que l'on réalise la biotransformation à un pH de 6 à 11 et à une température de 15 à 55°C.

12. ADN codant une hydrolase stéréospécifique caractérisé par la carte de restriction suivante (fig. 1):

```
                                    ┌── EcoRI  -0,45
                                    │
                                       EcoRV  -0,43


                                    ├── PvuII   0



                                    ├      0,29


                                    ├── KspI   0,50
                                    ├── SmaI   0,55
                                    ├── PstI   0,56
                                    ├── SmaI   0,74




                                    ├── StuI   1,28

                                       SmaI   1,29


                                          1,57



                                    ├── PstI   1,81
                                       BamHI  1,85
```

13. Fragment d'ADN codant un polypeptide à activité d'hydrolase stéréospécifique dont la séquence d'acides aminés est représentée sur la figure 3.

14. Fragment d'ADN qui s'hybride avec le fragment d'ADN représenté sur la figure 3 et qui code un polypeptide à activité d'hydrolase stéréospécifique.

15. ADN ou fragment d'ADN selon la revendication 12, 13 ou 14 dans le plasmide hybride pCAR6 déposé dans Escherichia coli XL1-Blue (n° DSM 6551).

16. ADN ou fragment d'ADN selon la revendication 12, 13 ou 14 dans le plasmide hybride pCAR6 déposé dans Es-

23

cherichia coli DH5 (n° DSM 7053).

**17.** Plasmide hybride consistant en un vecteur d'expression avec insertion de l'ADN ou du fragment d'ADN selon la revendication 12, 13 ou 14.

**18.** Plasmide hybride pCAR6 consistant en l'ADN ou le fragment d'ADN selon la revendication 12, 13 ou 14 et le vecteur d'expression pBLUESCRIPT-KS+, déposé dans Escherichia coli XL1-Blue (n° DSM 6551).

**19.** Plasmide hybride pCAR6 consistant en l'ADN ou le fragment d'ADN selon la revendication 12, 13 ou 14 et le vecteur d'expression pBLUESCRIPT-KS+, déposé dans Escherichia coli DH5 (n° DSM 7053).

**20.** Micro-organismes qui sont transformés avec un plasmide hybride selon la revendication 17, 18 ou 19.

**21.** Micro-organismes selon la revendication 20 de l'espèce Escherichia coli XL1-Blue (n° DSM 6551) qui sont transformés avec le plasmide hybride pCAR6.

**22.** Micro-organismes selon la revendication 20 de l'espèce Escherichia coli DH5 (n° DSM avec le plasmide hybride pCAR6.

Fig. 1

| | |
|---|---|
| EcoRI | -0,45 |
| EcoRV | -0,43 |
| PvuII | 0 |
| | 0,29 |
| KspI | 0,50 |
| SmaI | 0,55 |
| PstI | 0,66 |
| SmaI | 0,74 |
| StuI | 1,28 |
| SmaI | 1,29 |
| | 1,57 |
| PstI | 1,81 |
| BamHI | 1,85 |

Fig. 2

Fig. 3

PvuII
c agc tgc ggt tgc agg cca gcc gcc agc gcc tga tgc agg ggc

gct gct cca gcg tga ccg agg cgg cct tcg cgc atg ggt tct ccg atg cgg cgc act tca gcc gag cct ttc gca agg cgt tcg gct gca

cgc ccc gca gcc tgc tgg ccg cct gag caa ggt cgc tga cct cac gaa caa gcg tcg gcg ccg ccg gct ccc tag cat ggg cct gcc gtg

cgt ccc gtg cgg ctt ttc cag tgc aac aac ggt ggc gcc ccg gag ccg ggc gcc ggg aga cat gcc ATG AAT GAC AGC GAA CTG CAC CAT
                                                                                        MET Asn Asp Ser Glu Leu His His

CTC GAA CTG CTG GAG GTG GGG CGC GAG ATC CAG TCC CGC CGC ATT TCG TCG GAA GAG GTG ACC CGC CAC ATG CTG GCG CGC ATC GAG GCC
Leu Glu Leu Leu Glu Val Gly Arg Glu Ile Gln Ser Arg Arg Ile Ser Ser Glu Glu Val Thr Arg His MET Leu Ala Arg Ile Glu Ala

GTG GAC GCG CGG CTG CAC AGC TAC GTG ACG GTG ATG GCG CAG CAG GCG ATG GAG GAC GCC CGC CGC GCC GAC GCC GAG ATC GCG CAG GGC
Val Asp Ala Arg Leu His Ser Tyr Val Thr Val MET Ala Gln Gln Ala MET Glu Asp Ala Arg Arg Ala Asp Ala Glu Ile Ala Gln Gly
        KspI                                                              SmaI
GCC GCC GCG GTG CGC TGC ACG GCG TGC CGT GGC GCT CAA GGA CCT GCT GTG GAC CCG GGG CGT CCC CAC CAC GCA TGG AAT GAC GCT GCA
Ala Ala Ala Val Arg Cys Thr Ala Cys Arg Gly Ala Gln Gly Pro Ala Val Asp Pro Gly Arg Pro His His Ala Trp Asn Asp Ala Ala
                                                                                                        PstI
CCG CGA CCA TCG CCC CAC GGA AGA TGC CAC CGT GGT GCG CAG GCT GCG CGA GGC CGG CGC CGT CAT CCT GGG CAA GCT GCA GCA GAC CGA
Pro Arg Pro Ser Pro His Gly Arg Cys His Arg Gly Ala Gln Ala Ala Arg Gly Arg Arg Arg His Pro Gly Gln Ala Ala Ala Asp Arg
                                                                                        SmaI
AGG CGC CTT CGC CGA CCA CCA TCC CGA GAT CAC TGC CCC CGT CAA CCC CTG GAG CGC CCA GCT ATG GCC CGG GGC CTC GTC CAG CGG CTC
Arg Arg Leu Arg Arg Pro Pro Ser Arg Asp His Cys Pro Arg Gln Pro Leu Glu Arg Pro Ala MET Ala Arg Gly Leu Val Gln Arg Leu

GGG CGT GGC CAC GGC GGC GGG GCT GTG CTT CGG ATC GCT GGG CAC GGA CAC CGG GGG CTC CAT CCG CTT TCC ATC GGC CGC CAA CGG CAT
Gly Arg Gly His Gly Gly Gly Ala Val Leu Arg Ile Ala Gly His Gly His Arg Gly Leu His Pro Leu Ser Ile Gly Arg Gln Arg His

CAC GGG GCT CAA GCC CAC CTG GGG CAG GGT GAG CCG CCA CGG CGC CTT CGA ACT GGC CGC GTC CCT GGA CCA CAT AGG CCC GAT GGC GCG
His Gly Ala Gln Ala His Leu Gly Gln Gly Glu Pro Pro Arg Arg Leu Arg Thr Gly Arg Val Pro Gly Pro His Arg Pro Asp Gly Ala

CAG TGC TGC CGA TGC CGC AGC CAT GCT CGC GGC CAT CGC CGG CGG GGA CCC GCT GGA CCC TAC GGC CAG CCA GTG CAG CGT GCC CGA CTA
Gln Cys Cys Arg Cys Arg Ser His Ala Arg Gly His Arg Arg Gly Gly Pro Ala Gly Pro Tyr Gly Gln Pro Val Gln Arg Ala Arg Leu

TCT GGC CAT GAT GAC GCG CGG ATT CTC CGG CCT GCG CCT GGG CAT GGA CCG GCA ATG GGC ACT GGA CGG CGT GGA TGC CCC CTC CCG CCA
Ser Gly His Asp Asp Ala Arg Ile Leu Arg Pro Ala Pro Gly His Gly Pro Ala MET Gly Thr Gly Arg Arg Gly Cys Pro Leu Pro Pro

GGC GGT GGA GCA GGC CCT GGC GGT GGC GCA GCG CCT GGG GGC CAG CGT GCA GGA GGT CCG CTT TCC CGA TGC CAC CCA GGC GGT GGA GGA
Gly Gly Gly Ala Gly Pro Gly Gly Gly Ala Ala Pro Gly Gly Gln Arg Ala Gly Gly Pro Leu Ser Arg Cys His Pro Gly Gly Gly Gly
                                                                                        StuI        SmaI
CTG GCC GGC GCT GTG CGC GGT GGA GAC CGC CGT GGC GCA CGG CGC CAC GTT CCC TGC ACG GCG CGA GGC CTA TGG CCC CGG GCT CGC CGG
Leu Ala Gly Ala Val Arg Gly Gly Asp Arg Arg Gly Ala Arg Arg His Val Pro Cys Thr Ala Arg Gly Leu Trp Pro Arg Ala Arg Arg

GTT GAT CGA CCT GGG GCT GGG CCT GTC CGC CAC CGA CTA CCA GCG GCT GCT GCT GCG CCG CGC GGA CTT CAC GGG CCG GGT GCG TGC ACT
Val Asp Arg Pro Gly Ala Gly Pro Val Arg His Arg Leu Pro Ala Ala Ala Ala Ala Pro Arg Gly Leu His Gly Pro Gly Ala Cys Thr

CTT CGC GCA GGT GGA TCT GCT GCT GGT CCC CGC CAC GGC CTT TGC GGC CCC CAC GCT GCA ACG CAT GGC GCA TTT CGG CTC CGA TGC CGA
Leu Arg Ala Gly Gly Ser Ala Ala Gly Pro Arg His Gly Leu Cys Gly Pro His Ala Ala Thr His Gly Ala Phe Arg Leu Arg Cys Arg

GCT GTT CTC GGG CAT GCT GCG CTA CAC CTG CCC GTT CGA CCT CAC GGG CAG CCC CAC GAT CAC GCT GCC CGG CGG ACG CAC TTC TGA ggg
Ala Val Leu Gly His Ala Ala Leu His Leu Pro Val Arg Pro His Gly Gln Pro His Asp His Ala Ala Arg Arg Thr His Phe ---

cgc gcc cgt ggc ctt cca gtt cgt ggc ccc cga ctt ccg cga aga cct gct ggt gcg cgc ggg ctg ggc gtt cca gca ggc cac gga ctc

gca cag aca gca ccc tgc tgc ctg agc tgc ctg agc cgc cag gcc ggt ggc gcg aca cgg gcc tgt cac aca gcc ttc cta gac tgg cgt

                                                                                PstI                    BamHI
gat gtc ctt gat cga gat gga agg tgt cgc caa gtc ctg ggg cgg cac cac ggc gct gca ggc gct gga tct gcg cat tga acc cgg atc

c